# FASCICULE DE BREVET EUROPEEN

(11) **EP 1 343 432 B1**
(45) Date de publication et mention de la délivrance du brevet: **12.12.2007**
(21) Numéro de dépôt: 01995768.7
(22) Date de dépôt: 21.12.2001
(51) Int. Cl.: A61F 2/00

(54) **IMPLANT PROTHETIQUE POUR ORIFICE ANATOMIQUE OU CHIRURGICAL**
IMPLANTAT FÜR ANATOMISCHE ODER CHIRUGISCHE ÖFFNUNG
PROSTHETIC IMPLANT FOR ANATOMICAL OR SURGICAL ORIFICE

(30) Priorité: 22.12.2000 FR 0016942
(43) Date de publication de la demande: 17.09.2003
(73) Titulaire: Cousin Biotech (S.A.S.), 59117 Wervicq Sud (FR)
(72) Inventeur: LEROY, Joel, F-62160 Bully les Mines (FR); SOLECKI, Gilles, F-59390 Lys-lez-Lannoy (FR)
(74) Mandataire: Thinat, Michel
(86) Numéro de dépôt international: PCT/FR2001/004168
(87) Numéro de publication internationale: WO 2002/051334

(56) Documents cités:
- EP-A- 0 744 162
- EP-A- 0 888 756
- WO-A-99/03422
- FR-A- 2 735 353
- FR-A- 2 767 672
- FR-A- 2 769 825
- US-A- 4 693 720

## Description

La présente invention a pour objet un implant prothétique ou obturateur pour orifice anatomique ou chirurgical destiné en particulier, mais non exclusivement, à l'obturation des orifices de la paroi musculaire. Ces orifices proviennent d'un affaiblissement musculaire consécutif à une maladie, à un vieillissement des structures anatomiques, ou suite à une intervention chirurgicale.

Dans la paroi abdominale musculaire, lorsqu'un orifice est créé ou existe congénitalement il peut être source d'une herniation du contenu de la cavité abdominale. Sous l'effet de la pression abdominale les viscères peuvent alors être expulsés et incarcérés dans l'orifice herniaire avec parfois un étranglement.

Afin de résoudre ce type de problème, de nombreuses techniques ont été proposées qui ont pour but d'obturer l'orifice et de renforcer la paroi musculaire qui doit résister à la pression abdominale au niveau de l'orifice herniaire réparé. Cette pression, dépendant du contenu da la cavité abdominale et des contractions musculaires, est un des facteurs de récidive herniaire.

L'obturation de l'orifice herniaire par rapprochement des berges est la plus simple des techniques chirurgicales, mais elle présente un risque de section des berges par une traction excessive entraînant une récidive du défaut.

L'obturation de l'orifice par un tissu prothétique résistant permet un renforcement de la paroi musculaire. Le tissu peut être disposé en arrière de l'orifice herniaire ou en avant de l'orifice musculaire abdominal. Que ce soit en arrière ou en avant de l'orifice herniaire, la pièce prothétique doit déborder largement l'orifice pour être efficace.

La dissection des espaces en avant et/ou en arrière de l'orifice herniaire nécessite un délabrement des plans musculaires qui peut être source de complications (lésions nerveuses, vasculaires, hématomes).

Une autre solution connue consiste à réaliser l'obturation de l'orifice à l'aide d'un bouchon prothétique ou "plug". Le plug a pour but d'empêcher l'extériorisation du sac herniaire en le maintenant en profondeur derrière le plan postérieur musculaire. Ces bouchons sont soit incompressibles (LICHTENSTEIN), soit compressibles, (RUTKOW et ROBBINS). Les bouchons compressibles déformables doivent, pour être efficaces, être volumineux. Ils ont pour but d'obturer l'orifice et de créer une fibrose en arrière de l'orifice herniaire. Ils sont introduits dans l'orifice puis fixés sur les berges de celui-ci. L'écrasement et l'étalement progressif du plug conique, de part et d'autre des berges postérieures de l'orifice herniaire entraîne une obturation, puis une sclérose des tissus qui renforcent la paroi musculaire. La nécessité d'un volume important résulte de ce qu'il faut éviter le retournement du bouchon sous l'effet de la pression abdominale postérieure. Le volume du tissu prothétique non résorbable explique certaines complications décrites de douleurs, d'ulcérations d'organes de voisinage (vaisseaux, vessie). En raison du volume du plug, la fibrose résiduelle est importante et peut gêner un geste chirurgical ultérieur, notamment dans les hernies inguinales au niveau des gros vaisseaux iliaques sous-jacents.

FR-A-2735353 concerne une plaque très souple présentant une mémoire élastique importante réalisée en un tissu à mailles ajourées imprégné d'un élastomère ou plastomère en très faible quantité, pouvant être conformée en trois dimensions. Mais cette plaque reste souple.

WO 9903422 divulgue une prothèse en forme de disque de réparation de tissus musculaires, comprenant des couches multiples de tricots. Cette prothèse épouse la forme du défaut à combler.

FR-2 767 672 décrit une prothèse destinée à obturer un canal herniaire comprenant deux parties constituant respectivement le corps de l'obturateur et un anneau de forme torique entourant la première partie. La tête enflée s'oppose élastiquement à la pression interne et s'écrase sur la partie torique de manière à ce que l'effort exercé soit réparti sur une portion importante de la cavité.

Un premier objet de l'invention est de pallier les inconvénients des obturateurs connus et de proposer un obturateur rigide, résorbable, peu volumineux, résistant à la pression abdominale postérieure et provoquant une sclérose à l'origine de l'obturation de l'orifice herniaire. La résorption peut être totale ou partielle dans l'espace et dans le temps. L'association à une prothèse non résorbable fixée en avant du plan musculaire selon la technique décrite par Lichtenstein renforce définitivement la paroi musculaire et évite donc le risque de récidive herniaire à distance.

Selon l'invention, l'obturateur pour orifice anatomique ou chirurgical, destiné à venir en appui sur la face interne de l'orifice, comprenant, après pose, une partie interne à l'orifice et une partie externe appliquée contre le bord de l'orifice est caractérisé en ce que son matériau constitutif est rigide (ou rigidifié avant la pose), la partie interne présentant une forme ogivale dont le sommet est orienté vers l'intérieur de l'organisme, la partie externe constituant une base circulaire fixée aux berges de l'orifice.

Selon une autre caractéristique de l'invention, l'obturateur est réalisé en un matériau résorbable biocompatible à bords et surfaces externes atraumatiques.

Pour contrebalancer la pression interne des viscères, la présente invention propose de former, en regard de ceux-ci une surface convexe pouvant s'aplatir ce qui accroît son ancrage à l'intérieur de l'orifice. Cette surface convexe peut être constitué par une demi sphère, une cupule, un dôme ou plus généralement une ogive. La structure du bouchon est telle qu'elle résiste, par elle-même, sans se retourner, à une pression au moins deux fois supérieure à la pression abdominale maximale.

D'autres caractéristiques et avantages de l'invention apparaîtront au cours de la description qui va suivre d'un mode particulier de réalisation, donné uniquement à titre d'exemple non limitatif, en regard des dessins qui représentent :
- La figure 1, un schéma montrant le développement d'une hernie que l'invention vise à résorber;
- la figure 2, une vue en perspective d'un plug selon l'invention;
- la figure 2a, un mode de fabrication d'un plug selon l'invention;
- la figure 3, une vue par dessous du même plug;
- la figure 4, une vue en coupe de la prothèse en position dans l'orifice herniaire;
- la figure 5, une deuxième forme du plug;
- la figure 6, une vue en coupe de la seconde prothèse en position dans un orifice herniaire;
- la figure 7, un autre mode de réalisation du plug;
- la figure 8, encore un autre mode de réalisation le plug étant inséré dans un orifice herniaire;
- La figure 9, une variante de réalisation;
- la figure 10, un bouchon solidaire d'une jupe de fixation;
- la figure 11 un autre mode de réalisation dans lequel le plug présente des fentes latérales;
- la figure 12, un plug en forme de champignon.

Sur l'ensemble des figures, les mêmes références désignent les mêmes éléments.

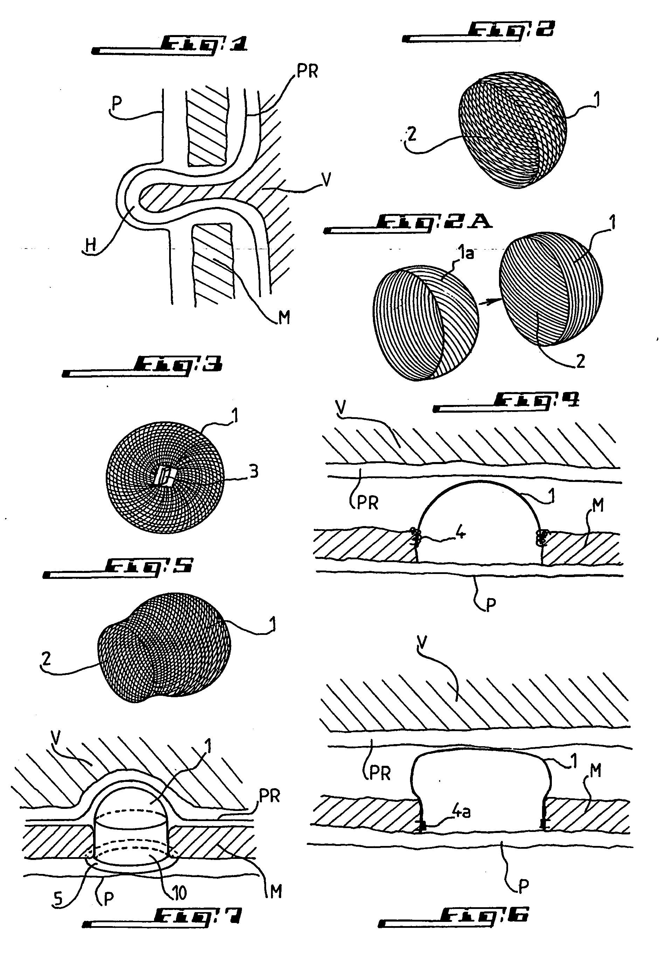

La figure 1 est un schéma permettant d'expliquer le problème à résoudre. Il représente, de la gauche vers la droite, du dessin, une protubérance herniaire H qui fait saillie au-dessous de la peau P. Cette protubérance provient d'un passage des viscères V, derrière le péritoine PR, à travers une faille de la paroi abdominale M constituée de muscles et de graisse dans des épaisseurs variables. Pour réparer la paroi abdominale dans la région herniaire, on implante dans l'organisme une prothèse pouvant résister à la pression des viscères V.

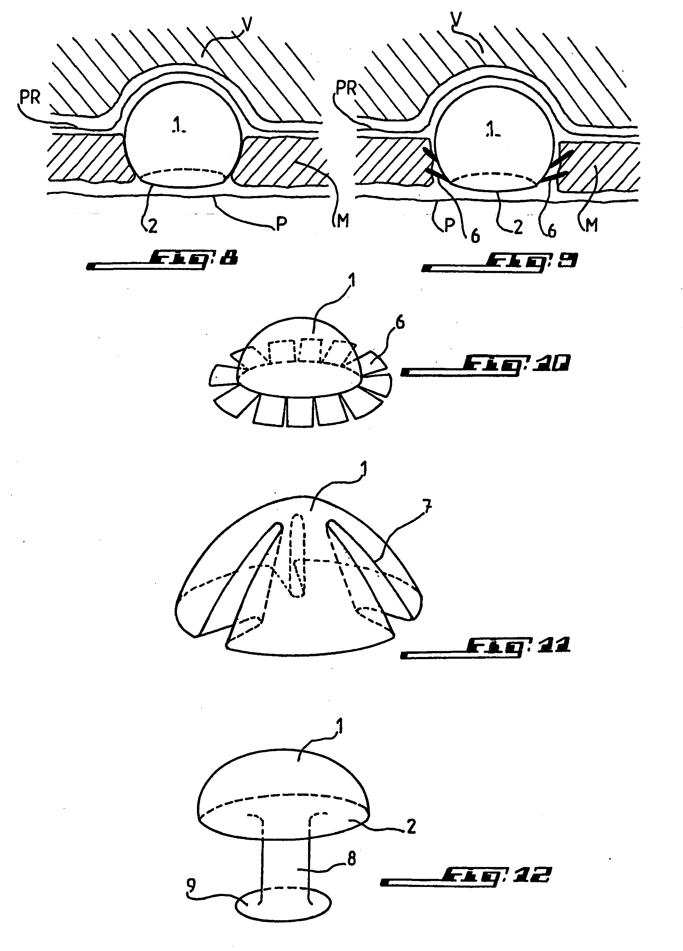

Sur la figure 2, qui est une vue en perspective d'un plug, on voit que le corps de la prothèse atraumatique présente la forme d'une demi sphère creuse 1 dont la base 2 est ouverte. Pour obturer l'orifice herniaire, la partie ogivale 1 de la cupule est introduite dans l'orifice, de l'extérieur vers l'intérieur et poussée à l'aide d'un manche (non représenté) pour pénétrer plus ou moins profondément dans l'orifice. Après quoi, la base 2 qui présente, de préférence, un bord multiperforé et/ou une collerette de fixation, est solidarisée, par exemple par suture ou agrafage, avec les berges de l'orifice. Pour que l'obturation se produise convenablement, le plug 1 doit être rigide ou rigidifié lors de sa pose.

La figure 2A représente un moyen permettant d'obtenir une telle rigidité qui consiste à assembler plusieurs demi-sphères (seulement deux étant représentées sur la figure 2A) enfilées les unes dans les autres et assemblées par exemple par collage. Le procédé de fabrication de la forme ogivale du plug peut être le tricotage, le tissage ou tout autre procédé permettant d'assembler des fibres textiles. Le procédé de fabrication peut faire appel à l'injection de plastique.

Comme cela apparaît mieux sur la figure 3, la partie concave du dôme est creuse et comporte un moyen 3 tel que fil ou languette qui permet de saisir, d'introduire et de situer l'obturateur dans sa cavité.

La figure 4 représente en coupe un bouchon selon l'invention en position à l'intérieur de l'organisme. Le dôme 1 est fixé par son bord inférieur par une suture périphérique 4 sur les berges de l'orifice.

La figure 5 représente, en perspective, une autre mode de réalisation selon l'invention, dans lequel l'implant présente la forme d'une ampoule c'est-à-dire que le diamètre de la partie convexe 1 est supérieur au diamètre de l'ouverture inférieure 2.

La figure 6 représente en coupe un obturateur, du type représenté sur la figure 5, fixé dans un orifice herniaire. La partie inférieure de l'implant formant jupe est fixée sur les berges de l'orifice par des agrafes 4a ou des fils de suture.

La figure 7 représente, dans les mêmes conditions un plug comprenant, comme précédemment une partie convexe 1 se prolongeant par une jupe cylindrique 10 dont la hauteur peut être adaptée à la hauteur de l'orifice. La jupe cylindrique peut présenter, à sa partie inférieure une collerette 5 fixée par tout moyen sur la surface extérieure de la paroi abdominale M.

L'obturateur peut également, comme représenté sur la figure 8, avoir une forme sphérique, la sphère étant complète ou tronquée. Il est enfoncé dans l'orifice jusqu'à ce que sa partie de plus grand diamètre dépasse le niveau de la face intérieure de la paroi abdominale M. De préférence, les obturateurs présentent des languettes d'ancrage 6 qui, comme représenté sur la figure 9, empêchent le mouvement du plug en direction du péritoine PR.

La figure 10 représente un obturateur à surface supérieure convexe 1 dont le bord inférieur 2 est entouré de languettes d'accrochage 6. Ces languettes prennent appui contre la surface interne de la paroi abdominale M après l'introduction de l'obturateur dans l'orifice herniaire qui provoque le repliement temporaire des languettes.

Les figures 11 et 12 représentent deux autres modes de réalisation d'un plug ou bouchon obturateur.

Sur la figure 11, l'implant 1 présente trois plis 7 permettant un écrasement afin de faciliter l'introduction dans l'orifice de la hernie. Dans un autre mode de réalisation (non représenté), la paroi comprend des baleines rigide recouvertes d'une couche textile. Il est également possible de prévoir un renfort à la base de la demi sphère.

Sur la figure 12, l'implant présente une forme en champignon dont le sommet est hémisphérique et creux et qui se prolonge par une colonne tubulaire 8 se terminant vers le bas par une butée 9. L'implant est enfoncé à force dans l'orifice herniaire et la paroi ventrale M est immobilisée entre la partie inférieure du bouchon 1 et la butée.

Dans un autre mode de réalisation (non représenté), l'implant prothétique est constitué par une sphère (ou des boudins) de matériau résorbable remplie de sérum physiologique ou autre liquide biocompatible, la sphère étant introduite entre la paroi musculaire et le péritoine.

Le bouchon obturateur rigide selon l'invention est de préférence réalisé en un matériau résorbable à base d'acide polyglycolique ou de polymère d'acide lactique de forme L ou D ou de polymère d'acide polyglycolique et d'acide lactique.

La résorption lente du bouchon est variable dans l'espace et le temps. Elle est calculée pour entraîner un affaissement progressif de l'implant qui est remplacé par un tissu de sclérose. La demi-sphère peut être associée à une feuille prothétique non résorbable fixée sur la partie convexe ogivale. La feuille prothétique est introduite par l'orifice herniaire dans l'espace rétro-musculaire où elle est déployée. Le bouchon est alors fixé comme indiqué précédemment évitant un risque de migration externe de la prothèse.

Le bouchon peut être solidarisé avec une feuille prothétique non résorbable fixée sur la partie convexe du dôme. Dans ce cas, la feuille prothétique est introduite par l'orifice herniaire dans l'espace rétro musculaire où elle est déployée. Le dôme 1 est ensuite positionné dans l'orifice et fixé sur les berges de celui-ci ce qui évite un risque de migration externe du bouchon.

La demi sphère est composée de matière résorbable ou non résorbable ou partiellement résorbable. Cette demi sphère est, au départ, plus rigide que les plaques ou bouchon actuellement utilisés.

La demi sphère est, avantageusement, constituée de plusieurs couches, c'est-à-dire de plusieurs demi sphères collées les unes sur les autres ou assemblées par un autre procédé.

La demi sphère peut être obtenue en trempant une demi sphère textile non résorbable dans une solution constituée de solvant et de matière résorbable. Le résorbable utilisé est, par exemple, du PLLA, du PEA ou un copolymère de PLLA et PEA en proportions différentes (70/30 par exemple).

Il va de soi que de nombreuses variantes peuvent être apportées, notamment par substitution de moyens techniques équivalents, sans sortir pour cela du cadre de l'invention.

## Revendications

1. Obturateur prothétique biocompatible pour orifice anatomique ou chirurgical, destiné à venir en appui sur la face interne de l'orifice, **caractérisé en ce qu'**il comporte un corps de forme ogivale (1) terminé par une base circulaire (2), et **en ce qu'**il est réalisé en un matériau suffisamment rigide pour être introduit dans l'orifice sous cette forme ogivale de sorte qu'après la pose, le corps (1) forme partie interne (1) à l'orifice dont le sommet est orienté vers l'intérieur de l'organisme, et la base circulaire (2) forme partie externe (2) à l'orifice qui est fixée sur les bords de cet orifice.

2. Obturateur selon la revendication 1, **caractérisé en ce qu'**il est réalisé en un matériau textile résorbable biocompatible, tissé, tricoté ou non tissé, les fibres étant collées par fusion, à bords et surfaces externes atraumatiques.

3. Obturateur selon la revendication 1, **caractérisé en ce que** le corps (1) est hémisphérique.

4. Obturateur selon la revendication 1, **caractérisé en ce que** la base (2) est cylindrique et de diamètre inférieur à celui du corps (1).

5. Obturateur selon la revendication 1, **caractérisé en ce qu'**il est réalisé en un matériau au moins partiellement résorbable.

6. Obturateur selon la revendication 1, **caractérisé en ce que** la forme ogivale est obtenue par injection de matière plastique.

7. Obturateur selon la revendication 1, **caractérisé en ce que** le corps (1) est constitué de plusieurs couches (1, 1a) liées entre elles.

8. Obturateur selon la revendication 7, **caractérisé en ce que** les couches sont liées entre elles par un adhésif ou trempage dans un solvant.

9. Obturateur selon la revendication 5, **caractérisé en ce que** le matériau constitutif résorbable comprend de l'acide polyglycolique ou de polymère d'acide lactique de forme L ou D ou de polymère d'acide polyglycolique et d'acide lactique.

10. Obturateur selon la revendication 1, **caractérisé en ce qu'**une feuille prothétique est solidarisée du sommet du dôme (1).

## Claims

1. A biocompatible prosthetic obturator for an anatomic or surgical orifice, intended to press against the internal face of the orifice, **characterized in that** it includes an body (1) of ogival shape, terminated by a circular base (2), and **in that** it is made in a sufficiently stiff material for its introduction into the orifice in the form of this ogival shape, so that after it is put in, the body (1) forms a portion (1) internal to the orifice, the apex of which is oriented towards the inside of the organism, and the circular base (2) forms a portion (2) external to the orifice, which is attached onto the edges of this orifice.

2. The obturator according to claim 1, **characterized in that** it is made in a woven, knitted or non-woven biocompatible resorbable textile material, the fibers being melt-bonded, with atraumatic outer surfaces and edges.

3. The obturator according to claim 1, **characterized in that** the body (1) is hemispherical.

4. The obturator according to claim 1, **characterized in that** the base (2) is cylindrical and with a diameter less than that of the body (1).

5. The obturator according to claim 1, **characterized in that** it is made in an at least partially resorbable material.

6. The obturator according to claim 1, **characterized in that** the ogival shape is obtained by injection of plastic material.

7. The obturator according to claim 1, **characterized in that** the body (1) consists of several layers (1, 1a) bound together.

8. The obturator according to claim 7, **characterized in that** the layers are bound together by an adhesive or by soaking in a solvent.

9. The obturator according to claim 5, **characterized in that** the resorbable constitutive material comprises polyglycolic acid or a polymer of L- or D-form lactic acid or a polymer of polyglycolic acid and lactic acid.

10. The obturator according to claim 1, **characterized in that** a prosthetic sheet is made integral with the apex of the dome (1).

## Patentansprüche

1. Biologisch kompatibler prothetischer Verschluss für anatomische oder chirurgische Öffnung, der dazu bestimmt ist, sich auf der Innenseite der Öffnung abzustützen, **dadurch gekennzeichnet, dass** er einen Körper in spitzbogiger Form (1) umfasst, der in einer kreisförmigen Basis (2) abschließt, und **dadurch, dass** er aus einem ausreichend starren Werkstoff gefertigt ist, um in die Öffnung in dieser spitzbogigen Form einführbar zu sein, so dass der Körper (1) nach dem Einsetzen den Innenabschnitt (1) zur Öffnung bildet, dessen Gipfel in Richtung Innenraum des Organismus gerichtet ist, und die kreisförmige Basis (2) den Außenabschnitt (2) zur Öffnung bildet, der auf den Rändern dieser Öffnung befestigt ist.

2. Verschluss nach Anspruch 1, **dadurch gekennzeichnet, dass** er aus einem biologisch kompatiblen resorbierbaren textilen gewebten, gewirkten oder Vlies-Werkstoff gefertigt ist, wobei die Fasern durch Fusion geklebt sind, mit atraumatischen externen Rändern und Oberflächen.

3. Verschluss nach Anspruch 1, **dadurch gekennzeichnet, dass** der Körper (1) halbkugelförmig ist.

4. Verschluss nach Anspruch 1, **dadurch gekennzeichnet, dass** die Basis (2) zylindrisch ist und einen kleineren Durchmesser als der Körper (1) hat.

5. Verschluss nach Anspruch 1, **dadurch gekennzeichnet, dass** er aus einem mindestens teilweise resorbierbaren Werkstoff gefertigt ist.

6. Verschluss nach Anspruch 1, **dadurch gekennzeichnet, dass** die spitzbogige Form durch Einspritzen von Kunststoff hergestellt ist.

7. Verschluss nach Anspruch 1, **dadurch gekennzeichnet, dass** der Körper (1) von mehreren untereinander verbundenen Schichten (1, 1a) gebildet wird.

8. Verschluss nach Anspruch 7, **dadurch gekennzeichnet, dass** die Schichten untereinander durch ein Haftmittel oder Eintauchen in ein Lösungsmittel verbunden sind.

9. Verschluss nach Anspruch 5, **dadurch gekennzeichnet, dass** der resorbierbare konstitutive Werkstoff Polyglycolsäure oder Milchsäurepolymer der L- oder D-Form oder Polyglycolsäure- und Milchsäurepolymer umfasst.

10. Verschluss nach Anspruch 1, **dadurch gekennzeichnet, dass** eine prothetische Folie mit dem Gipfel der Kuppel verbunden ist.
